(19) Europäisches Patentamt
European Patent Office
Office européen des brevets

(11) Veröffentlichungsnummer : **0 159 516**
**B1**

(12) **EUROPÄISCHE PATENTSCHRIFT**

(45) Veröffentlichungstag der Patentschrift : **20.01.88**

(51) Int. Cl.⁴ : **C 07 D291/06**

(21) Anmeldenummer : **85102884.5**

(22) Anmeldetag : **13.03.85**

(54) **Verfahren zur Herstellung von 6-Methyl-3,4-dihydro-1,2,3-oxathiazin-4-on-2,2-dioxid und dessen nicht-toxischen Salzen.**

(30) Priorität : **22.03.84 DE 3410440**

(43) Veröffentlichungstag der Anmeldung : **30.10.85 Patentblatt 85/44**

(45) Bekanntmachung des Hinweises auf die Patenterteilung : **20.01.88 Patentblatt 88/03**

(84) Benannte Vertragsstaaten : **AT BE CH DE FR GB IT LI NL SE**

(56) Entgegenhaltungen :
**DE-A- 2 001 017**
**DE-A- 2 228 423**
**DE-A- 2 327 804**
**DE-A- 2 434 549**
**DE-A- 2 434 564**
**CHEMICAL ABSTRACTS, vol. 86, no. 5, 31. Januar 1977, Columbus, Ohio, USA, K. CLAUSS, E. LUECK, G.W. VON RYMON LIPINSKI, "Acetosulfam, a new sweetener. 1. Synthesis amd properties", Seite 366, Zusammenfassung Nr. 29 776u**

(73) Patentinhaber : **HOECHST AKTIENGESELLSCHAFT**
**Postfach 80 03 20**
**D-6230 Frankfurt am Main 80 (DE)**

(72) Erfinder : **Clauss, Karl, Dr.**
**Im Birkenfeld 20**
**D-6233 Kelkheim (Taunus) (DE)**
Erfinder : **Linkies, Adolf, Dr.**
**Loreleistrasse 12**
**D-6230 Frankfurt am Main 80 (DE)**
Erfinder : **Reuschling, Dieter, Dr.**
**Beethovenstrasse 27**
**D-6308 Butzbach (DE)**

**EP 0 159 516 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

6-Methyl-3,4-dihydro-1,2,3-oxathiazin-4-on-2,2-dioxid ist die Verbindung der Formel

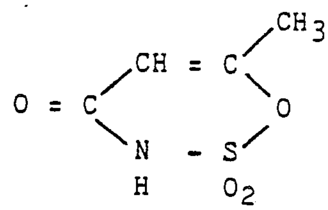

Infolge des aciden Wasserstoffs am Stickstoffatom ist die Verbindung zur Salzbildung (mit Basen) befähigt. Die nicht-toxischen Salze — wie z. B. das Na-, das K- und das Ca-Salz — können wegen ihres z. T. intensiven Süßgeschmacks als Süßstoffe auf dem Nahrungsmittelsektor verwendet werden, wobei das K-Salz (« Acesulfam K » oder auch nur « Acesulfam ») von besonderer Bedeutung ist.

Zur Herstellung des 6-Methyl-3,4-dihydro-1,2,3-oxathiazin-4-on-2,3-dioxids und dessen nicht-toxischer Salze ist eine Reihe verschiedener Verfahren bekannt ; vgl. Angewandte Chemie 85, Heft 22 (1973) S. 965 bis 73, entsprechend International Edition Vol. 12, No. 11 (1973), S. 869-76. Praktisch alle Verfahren gehen von Chlor- oder Fluorsulfonylisocyanat ($XSO_2NCO$ mit X = Cl oder F) aus. Das Chlor- bzw. Fluorsulfonylisocyanat wird dann mit Monomethylacetylen, Aceton, Acetessigsäure, Acetessigsäure-tert.-butylester oder Benzylpropenylether (in einer meist mehrstufigen Reaktion) zu Acetoacetamid-N-sulfochlorid bzw. -fluorid umgesetzt, was unter der Einwirkung von Basen (wie z. B. methanolischer KOH) cyclisiert und die entsprechenden Salze des 6-Methyl-3,4-dihydro-1,2,3-oxathiazin-4-on 2,2-dioxids liefert. Aus den Salzen kann das freie Oxathiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsulfonylisocyanats (DE-OS 2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperaturen zwischen etwa —30 und 100 °C umgesetzt ; die Umsetzung verläuft nach folgender Reaktionsgleichung (mit Triethylamin als Amin) :

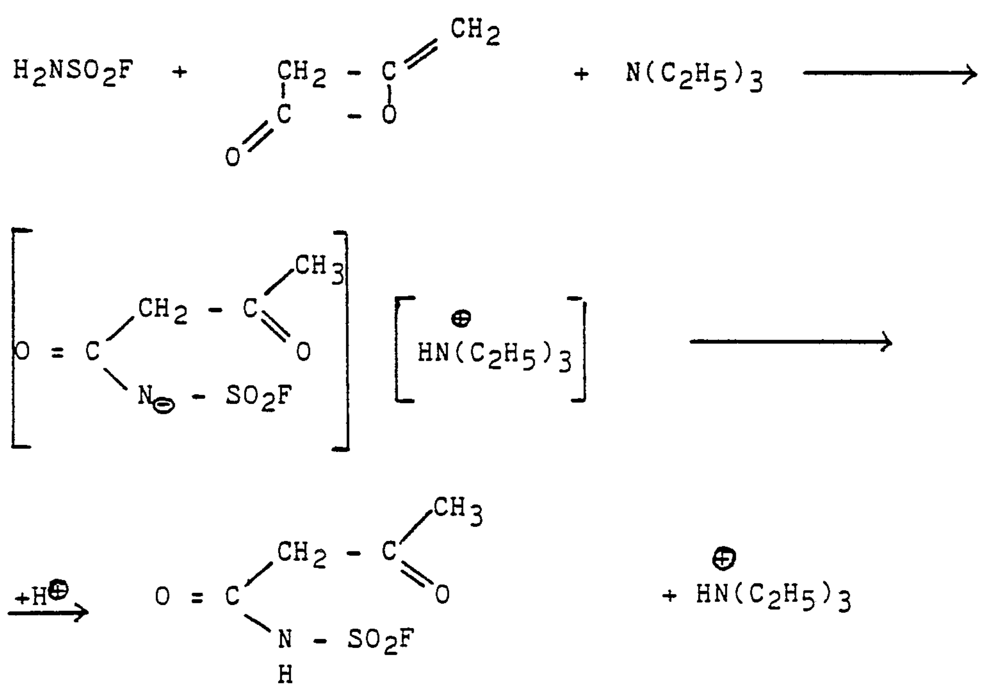


Acetoacetamid-N-sulfofluorid

Das Acetoacetamid-N-sulfofluorid wird dann auf übliche Weise mittels einer Base, z. B. mit methanolischer KOH, zum Süßstoff cyclisiert :

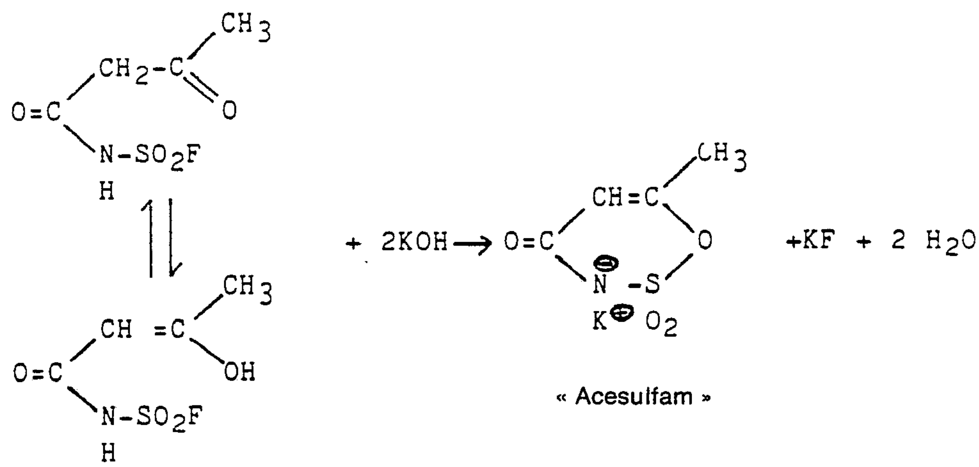

« Acesulfam »

Obwohl die bekannten Verfahren z. T. recht befriedigende Ausbeuten an 6-Methyl-3,4-dihydro-1,2,3-oxathiazin-4-on-2,2-dioxid und dessen nicht-toxischen Salzen liefern, (bis zu ca. 85 % d. Th., bezogen auf das Augangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig ; die Herstellung des Chlor- und Fluor-sulfonylisocyanats erfordert nämlich wegen der z. T. ziemlich unangenehm handzuhabenden Ausgangsmaterialien (HCN, $Cl_2$, $SO_3$ und HF) erhebliche Vorsichtsmaßnahmen und Sicherheitsvorkehrungen. Der Herstellung des Chlor- und Fluor-sulfonylisocyanats liegen folgende Reaktionsgleichungen zugrunde :

$$HCN + Cl_2 \rightarrow ClCN + HCl$$

$$ClCN + SO_3 \rightarrow ClSO_2NCO$$

$$ClSO_2NCO + HF \rightarrow FSO_2NCO + HCl$$

Der Ersatz des Amidosulfofluorids in dem Verfahren gemäß der vorerwähnten DE-OS 24 53 063 etwa durch die wesentlich leichter (z. B. aus $NH_3 + SO_3$) erhältliche Amidosulfonsäure $H_2NSO_3H$ bzw. deren Salze erschien kaum erfolgversprechend, weil nämlich die Umsetzung des Na-Amidosulfonats $H_2NSO_3Na$ mit Diketen in wässrig-alkalischer Lösung überhaupt kein rein isolierbares Umsetzungsprodukt ergibt. Das bei dieser Umsetzung wohl zumindest mit entstandene 1 : 1-Addukt konnte vielmehr nur in Form des Kupplungsproduktes mit 4-Nitrophenyldiazoniumchlorid als blaßgelber Farbstoff gewonnen werden ; vgl. Ber. 83 (1950), S. 551-558, insbesondere S. 555, letzter Absatz vor der Beschreibung der Versuche und S. 558, letzter Absatz :

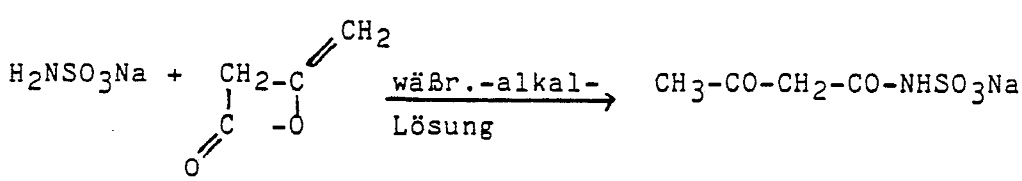


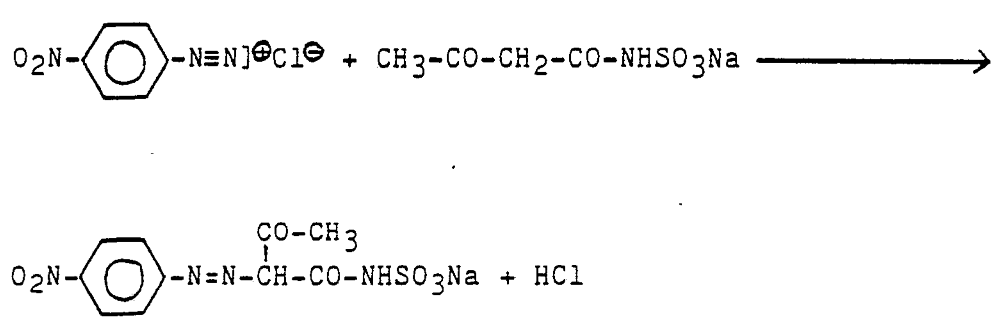

Die Acetoacetamid-N-sulfonsäure ist im übrigen ansonsten nur bzw. auch als Zwischenprodukt bei der Zersetzung des 6-Methyl-3,4-dihydro-1,2,3-oxathiazin-4-on-2,2-dioxids während des Kochens in wässriger Lösung postuliert worden ; vgl. die anfangs zitierte Literatur Angew. Chemie (1973) a.a.O. :

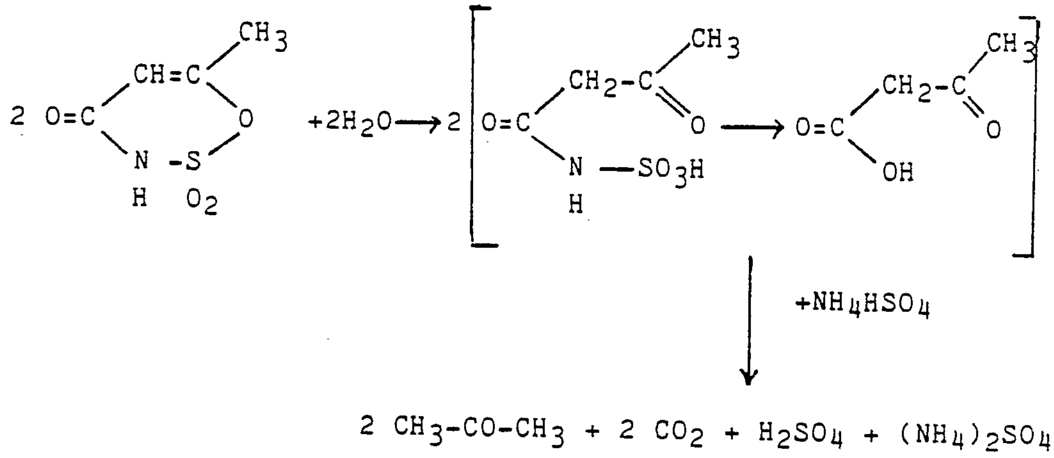


Wegen der insbesondere infolge der Notwendigkeit des Einsatzes nicht ganz einfach zugänglicher Ausgangsstoffe vor allem für die Durchführung in technischem Maßstab nicht ganz befriedigenden Verfahren des Standes der Technik zur Herstellung von 6-Methyl-3,4-dihydro-1,2,3-oxathiazin-4-on-2,2-dioxid und dessen nicht-toxischer Salze bestand somit die Aufgabe, die bekannten Verfahren entsprechend zu verbessern oder ein neues verbessertes Verfahren zu entwickeln.

Diese Aufgabe konnte erfindungsgemäß durch die Umsetzung von Acetoacetamid mit der mindestens etwa 2-molaren Menge $SO_3$ gelöst werden.

Erfindungsgegenstand ist daher ein Verfahren zur Herstellung von 6-Methyl-3,4-dihydro-1,2,3-oxathiazin-4-on-2,2-dioxid und dessen nicht toxischen Salzen, ausgehend von einer Acetoacetylverbindung ; das Verfahren ist dadurch gekennzeichnet, daß man Acetoacetamid mit der mindestens 2-molaren Menge $SO_3$, gegebenenfalls in einem inerten anorganischen oder organischen Lösungsmittel, umsetzt und daß man das dabei in der Säureform gebildete 6-Methyl-3,4-dihydro-1,2,3-oxathiazin-4-on-2,2-dioxid gegebenenfalls dann noch mit einer Base neutralisiert.

Bei der Umsetzung entsteht wahrscheinlich zuerst aus einem Mol Acetoacetamid und einem Mol $SO_3$ die Acetacetoamid-N-sulfonsäure, die dann mit einem weiteren Mol $SO_3$ zu dem 6-Methyl-3,4-dihydro-1,2,3-oxathiaziñ-4-on-2,2-dioxid cyclisiert :

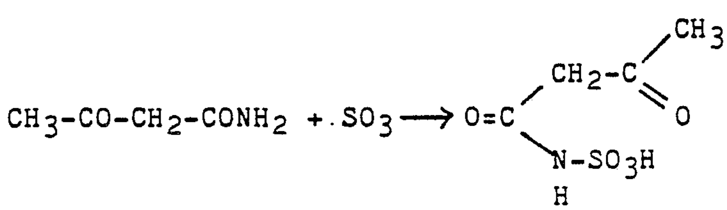

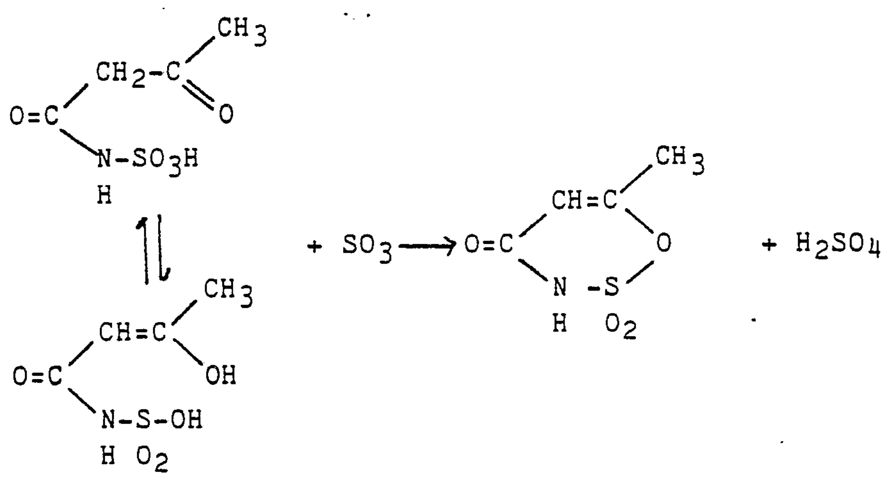

Dabei werden Ausbeuten von etwa 30 bis zu etwa 90 % d. Th., bezogen auf das Acetoacetamid, erhalten. Insbesondere wegen der einfachen und wohlfeilen Ausgangsstoffe sowie der außerordentlich einfachen Durchführbarkeit der Reaktion stellt das Verfahren einen erheblichen Fortschritt aud diesem Gebiet dar.

Das Gelingen der Umsetzung — insbesondere der Ringschlußreaktion — war sehr überraschend, weil nämlich die Cyclisierung — bei der im Ergebnis ein Mol Wasser pro Mol Acetoacetamid-N-sulfonsäure abgespalten wird — mit anderen wasserasbpaltenden Mitteln wie z. B. $P_2O_5$, Acetanhydrid, Trifluoressigsäureanhydrid, Thionylchlorid etc. nicht oder jedenfalls praktisch nicht gelingt.

Acetoacetamid ist z. B. aus Acetoacetylchlorid oder Diketen und $NH_3$ erhältlich und ist im übrigen auch ein gängiges Handelsprodukt.

Das Acetoacetamid wird dann mit der mindestens 2-molaren Menge $SO_3$ (pro Mol Acetoacetamid) umgesetzt. Vorzugsweise beträgt die $SO_3$-Menge 2 bis 20 Mol, insbesondere 4 bis 10 Mol pro Mol Acetoacetamid. Es kann dem Reaktionsansatz sowohl in fester oder flüssiger Form als auch durch Einkondensation von $SO_3$-Dampf zugegeben werden.

Die üblichere Zugabeform besteht jedoch in der Zugabe einer $SO_3$-Lösung in konzentrierter Schwefelsäure, flüssigem $SO_2$ oder einem inerten organischen Lösungsmittel.

Auch der Einsatz von $SO_3$-abspaltenden reaktiven $SO_3$-Derivaten, ist möglich. Besonders günstig auf den Reaktionsverlauf wirkt sich der teilweise Ersatz des freien $SO_3$ durch ein reaktives $SO_3$-Derivat aus. Solche reaktiven $SO_3$-Derivate sind z. B. Addukte von $SO_3$ an tertiäre Amine oder an N-alkylsubstituierte Carbonsäureamide, vorzugsweise an solche tertiären Amine, bei denen auf ein N-Atom bis zu 20, insbesondere nur bis zu 10 C-Atome kommen. Folgende tertiäre Amine sind in beispielhafter Weise zu nennen :

Trimethylamin, Triethylamin, Tri-n-propylamin, Triisopropylamin, Tri-n-butylamin, Triisobutylamin, Tricyclohexylamin, Ethyldiisopropylamin, Etehyldicyclohexylamin, N,N-Dimethylanilin, N,N-Dimethylanilin, Benzyldimethylamin, Pyridin, substituierte Pyridine wie Picoline, Lutidine, Collidine oder Methylethylpyridine, N-Methylpiperidin, N-Ethylpiperidin, N-Methylmorpholin, N,N-dimethylpiperazin, 1,5-Diazabicyclo-[4.3.0]-nonen-(5), 1,8-Diazabicyclo-[5.4.0]-undecen-(7), ferner Tetramethylhexamethylendiamin, Tetramethylethylendiamin, Tetramethylpropylendiamin, Tetramethylbutylendiamin, oder auch 1,2-Dimorpholylethan, Pentamethyldiethylentriamin, Pentaethyldiethylentriamin, Pentamethyldipropylentriamin, Tetramethyldiaminomethan, Tetrapropyldiaminomethan, Hexamethyltriethylentetramin, Hexamethyltripropylentetramin, Diisobutylentriamin oder Triisopropylentetramin.

Besonders günstige reaktive $SO_3$-Derivative sind :

$(CH_3)_3N \cdot SO_3$, $(C_2H_5)_3N \cdot SO_3$, Pyridin $\cdot$ $SO_3$, 2-Picolin $\cdot$ $SO_3$, 2,6-Lutidin $\cdot$ $SO_3$ und Collidin $\cdot$ $SO_3$. Auch etwa das Addukt $HCON(CH_3)_2 \cdot SO_3$ ist mit Erfolg einsetzbar.

Die Addukte können in situ erzeugt werden.

Die erfindungsgemäße Umsetzung kann zwar im Prinzip ohne Lösungsmittel durchgeführt werden, doch ist die Durchführung in einem inerten anorganischen oder organischen Lösungsmittel bevorzugt. Als solche inerten anorganischen oder organischen Lösungsmittel kommen Flüssigkeiten in Frage, die mit $SO_3$ bzw. dessen reaktiven Derivaten sowie mit Acetoacetamid und dem Reaktionsendprodukt nich in unerwünschter Weise reagieren. Wegen der erheblichen Reaktionsfähigkeit insbesondere des $SO_3$ und dessen reaktiver Addukte, kommen daher hier nur relativ wenige Lösungsmittel in Frage. Bevorzugte Lösungsmittel sind :

Anorganische Lösungsmittel : flüssiges $SO_2$ ;

organische Lösungsmittel :

halogenierte aliphatische Kohlenwasserstoffe, vorzugsweise mit bis zu 4 C-Atomen wie z. B. Methylenchlorid, Chloroform, 1,2-Dichlorethan, Trichlorethylen, Tetrachlorethylen und Trichlor-fluorethylen ;

Kohlensäureester mit niederen aliphatischen Alkoholen, vorzugsweise mit Methanol oder Ethanol ;

Nitroalkane, vorzugsweise mit bis zu 4 C-Atomen, insbesondere Nitromethan ;

Pyridin und alkylsubstituierte Pyridine, vorzugsweise Collidin ; und

aliphatische Sulfone, vorzugsweise Sulfolan.

Die organischen Lösungsmittel können sowohl einzeln als auch in Mischung eingesetzt werden.

Von den organischen Lösungsmitteln besonders bevorzugt sind : Methylenchlorid, Chloroform, 1,2-Dichlorethan, Dimethylcarbonat, Nitromethan und Collidin.

Die Menge des eingesetzten inerten Lösungsmittels ist nicht kritisch. Wenn ein Lösungsmittel eingesetzt wird, soll lediglich eine ausreichende Lösung der Reaktanten gewährleist sein ; nach oben ist die Menge des Lösungsmittels von Wirtschaftlichkeitserwägungen begrenzt.

Die Reaktionstemperatur liegt normalerweise zwischen etwa — 70 und + 180 °C, vorzugsweise zwischen etwa — 40 und + 90 °C.

Die Reaktion wird normalerweise bei Atmosphärendruck durchgeführt.

Die Reaktionszeit kann zwischen einigen Minuten (bei höheren Temperaturen) bis zu einigen Tagen (im unteren Temperaturbereich) betragen.

Man kann die Reaktionsführung in der Weise gestalten, daß man das Acetoacetamid, gegebenenfalls in Lösung, vorlegt und $SO_3$ bzw. das reaktive $SO_3$-Addukt, gegebenenfalls in gelöster Form, zudosiert oder beide Reaktionspartner gleichzeitig in den Reaktionsraum schleust oder $SO_3$ bzw. dessen reaktive Derivate vorlegt und Acetoacetamid zuführt oder etwa Acetoacetamid zunächst mit ca. 1 bis 5 Mol, bevorzugt mit ca. 1 bis 2 Mol, eines reaktiven $SO_3$-Derivates (pro Mol Acetoacetamid) ca. 20 Minuten bis 48 Stunden, vorzugsweise ca. 30 Minuten bis 24 Stunden, bei etwa — 30 bis + 180 °C, vorzugsweise bei etwa 0 bis 90 °C, behandelt und diese Lösung zum $SO_3$ zudosiert.

Bevorzugt wird erst das Acetoacetamid mit einem reaktiven $SO_3$-Derivat umgesetzt. Anschließend wird ein Teil des $SO_3$ vorgelegt und dann entweder kontinuierlich oder portionsweise sowohl die Reaktionslösung aus Acetoacetamid und reaktivem $SO_3$-Derivat als auch $SO_3$ zudosiert.

Nach Beendigung der Reaktion wird der Ansatz normalerweise noch etwa 1/2 Stunde bis zu einigen Stunden nachgerührt.

Die Aufarbeitung des Reaktionsansatzes geschieht auf übliche Weise. Im Falle der Verwendung von (mit Wasser nicht mischbaren) inerten organischen Lösungsmitteln als Reaktionsmedium kann die Aufarbeitung beispielsweise wie folgt vorgenommen werden :

Man versetzt die $SO_3$-haltige Lösung mit der (bezogen auf $SO_3$) etwa 10-fachen molaren Menge Eis oder Wasser. Hierdurch wird eine Phasentrennung hervorgerufen : Das gebildete 6-Methyl-3,4-dihydro-1,2,3-oxathiazin-4-on-2,2-dioxid befindet sich hauptsächlich in der organischen Phase. Die noch in der wässrigen Schwefelsäure befindlichen Anteile können durch Extraktion mit einem organischen Lösungsmittel wie z. B. Methylenchlorid oder Essigester gewonnen werden. Die vereinigten organischen Phasen werden dann z. B. mit Natriumsulfat getrocknet und eingeengt. Falls die Gewinnung der freien Verbindung beabsichtigt ist, wird diese noch auf übliche Weise gereinigt (vorzugsweise durch Umkristallisation). Die Ausbeute liegt zwischen etwa 30 und 90 % d. Th., bezogen auf das Acetoacetamid.

Wenn jedoch die Gewinnung eines nicht-toxischen Salzes des 6-Methyl-3,4-dihydro-1,2,3-oxathiazin-4-on-2,2-dioxid beabsichtigt ist, folgt noch die Neutralisation mit einer Base. Zu diesem Zweck werden vorteilhaft die im Zuge der Aufarbeitung des Reaktionsansatzes vereinigten getrockneten und eingeengten organischen Phasen in geeigneten organischen Lösungsmitteln, wie z. B. Alkoholen, Ketonen, Estern oder Ethern oder auch in Wasser mit einer entsprechenden Base — vorzugsweise mit einer Kaliumbase wie z. B. KOH, $KHCO_3$, $K_2CO_3$, K-Alkoholate etc. — neutralisiert. Das Oxathiazinon-Salz fällt dann, gegebenenfalls nach Einengen der Lösung in kristalliner Form aus und kann zur Reinigung noch umkristallisiert werden.

Die Neutralisationsstufe verläuft mit praktisch 100 %iger Ausbeute.

Die folgenden Beispiele sollen der Weiteren Erläuterung der Erfindung dienen. Nach den (Erfindungs-) Beispielen folgt noch ein Vergleichsbeispiel, welches zeigt, daß Acetoacetamid-N-sulfonsäure mit anderen wasserabspaltenden Mittel als $SO_3$ — hier mit $P_2O_5$ — nicht cyclisiert.

## Beispiel 1

Zu 8 ml (200 mMol) flüssigem $SO_3$ in 50 ml $CH_2Cl_2$ wurden bei —60 °C 5,1 g (50 mMol) Acetoacetamid in 50 ml $CH_2Cl_2$ getropft. Nach 2 Stunden kamen 50 ml Ethylacetat und 50 ml Wasser zur Lösung. die organische Phase wurde abgetrennt, die wäßrige noch zweimal mit Ethylacetat extrahiert. Die vereinigten organischen Phasen wurden nach dem Trocknen über Natriumsulfat eingeengt und in Methanol gelöst. Beim Neutralisieren der Lösung mit methanolischer KOH fiel das Kaliumsalz des 6-Methyl-3,4-dihydro-1,2,3-oxathiazin-4-on-2,2-dioxids aus.

Ausbeute : 3,1 g = 31 %.

## Beispiel 2

15,9 g (100 mMol) des Pyridin · $SO_3$-Komplexes und 5,1 g (50 mMol) Acetoacetamid in 100 ml $CH_2Cl_2$ wurden 17 Stunden bei Raumtempratur gerührt. Der Ansatz wurde anschließend innerhalb von 10 Minuten bei —30 °C zu einer Lösung von 12 ml (300 mMol) $SO_3$ in 50 ml $CH_2Cl_2$ getropft. 20 Minuten später wurde wie in Beispiel 1 aufgearbeitet.

Ausbeute : 7,9 g = 79 %.

## Beispiel 3

Zu 13,2 ml (110 mMol) 2,4,6-Collidin in 50 ml $CH_2Cl_2$ wurde bei —40 °C eine Lösung von 4 ml (100 mMol) $SO_3$ in 20 ml $CH_2Cl_2$ getropft. Dann wurde die Lösung 23 Stunden mit 9,1 g (90 mMol) Acetoacetamid bei Raumtemperatur gerührt. Diese Lösung wurde bei —30 °C innerhalb einer Stunde zu 4,4 ml (110 mMol) $SO_3$ in 200 ml $CH_2Cl_2$ getropft. Gleichzeitig wurde nach 12, 24, 36, 48 Minuten jeweils 4,4 ml (110 mMol) $SO_3$ zugegeben. 20 Minuten später wurde wie in Beispiel 1 unter Zugabe von 90 ml $H_2O$ aufgearbeitet.

Ausbeute : 11,8 g = 65 %.

## Beispiel 4

Zu 15,2 ml (110 mMol) Triethylamin in 50 ml 1,2-Dichlorethan wurde bei —40 °C die Lösung von 4 ml (100 mMol) $SO_3$ in 20 ml $CH_2Cl_2$ getropft. Die Lösung wurde 4 Stunden mit 5,1 g (50 mMol) Acetoacetamid gekocht. Danach wurde sie abgekühlt und bei —30 °C innerhalb von 1 Stunde zu einer Lösung von 2,4 ml (60 mMol) $SO_3$ in 50 ml $CH_2Cl_2$ getropft. Gleichzietig wurden nach 12, 24, 36, 48 Minuten jeweils 2,4 ml (60 mMol) $SO_3$ zugegeben. 20 Minuten später wurde wie in Beispiel 1 aufgearbeitet.

Ausbeute : 1 g = 10 %.

## Beispiel 5

Zu 13,2 ml (110 mMol) 2,4,6-Collidin in 50 ml $CH_2Cl_2$ wurde bei — 40 °C eine Lösung von 4 ml (100 mMol) $SO_3$ in 20 ml $CH_2Cl_2$ getropft. Nach Zugabe von 5,1 g (50 mMol) Acetoacetamid wurde 17 Stunden bei Raumtempratur gerührt. Diese Lösung wurde bei — 30 °C innerhalb von einer Stunde zu einer Lösung von 2,4 ml (60 mMol) $SO_3$ in 50 ml $CH_2Cl_2$ getropft. Gleichzeitig wurden nach 12 24, 36, 48 Minuten jeweils 2,4 ml (60 mMol) $SO_3$ zugegeben. 20 Minuten später wurde wie in Beispiel 1 aufgearbeitet.

Ausbeute : 9 g = 90 %.

## Beispiel 6

Zu 8 ml (200 mMol) flüssigem $SO_3$ in 150 ml $CH_2Cl_2$ wurden innerhalb 60 Minuten bei — 25 °C eine Lösung von 5,1 g (50 mMol) Acetoacetamid und 6,9 ml Triethylamin in 100 ml $CH_2Cl_2$ getropft und 90 Minuten bei — 25 °C nachgerührt. Die Aufarbeitung erfolgte wie in Beispiel 1.

Ausbeute : 4,1 g = 41 %.

## Beispiel 7

Es wurde wie in Beispiel 6 gearbeitet, aber statt 8 ml (200 mMol) flüssigem $SO_3$ wurden 16 g (200 mMol) festes $SO_3$ eingestetzt.

Ausbeute : 3,7 g = 37 %.

## Beispiel 8

Zu einer Mischung von 15,5 ml (250 mMol $SO_3$) 65 %igem Oleum in 150 ml $CH_2Cl_2$ wurden bei — 25 °C innerhalb 30 Minuten eine Lösung von 5,1 g (50 mMol) Acetoacetamid in 100 ml $CH_2Cl_2$ getropft und 60 Minuten bei — 25 °C nachgerührt. Die Aufarbeitung erfolgte wie in Beispiel 1.

Ausbeute : 2,3 g = 23 %.

## Vergleichsbeispiel

35,42 g (250 mMol) $P_2O_5$ wurden in 250 ml $CH_2Cl_2$ vorgelegt. Bei — 25 °C wurden innerhalb von 60 Minuten 62,5 ml Acetoacetamid-N-sulfonsäure-Lösung in $CH_2Cl_2$ mit einem Gehalt von 0,05 Mol der Sulfonsäure (= 9 g) hinzugetropft. Nach weiteren 60 Minuten bei — 25 °C wurde wie in Beispiel 1 aufgearbeitet. Im Reaktionsprodukt konnte dünnschichtchromatographisch kein 6-Methyl-3,4-dihydro-1,2,3-oxathiazin-4-on-2,2-dioxid bzw. dessen Kaliumsalz nachgewiesen werden.

## Patentansprüche

1. Verfahren zur Herstellung von 6-Methyl-3,4-dihydro-1,2,3-oxathiazin-4-on-2,2-dioxid und dessen nichttoxischen Salzen, ausgehend von einer Acetoacetylverbindung, dadurch gekennzeichnet, daß man Acetoacetamid mit der mindestens 2-molaren Menge $SO_3$, gegebenenfalls in einem inerten anorganischen oder organischen Lösungsmittel, umsetzt und daß man das dabei in der Säureform gebildete 6-Methyl-3,4-dihydro-1,2,3-oxathiazin-4-on-2,2-dioxid gegebenenfalls noch mit einer Base neutralisiert.

2. Verfahren nach Anspruch 1, dadurch gekennzeichnet, daß man das $SO_3$ in einer Menge von 2 bis 20 Mol/Mol Acetoacetamid, vorzugsweise in einer Menge von 4 bis 10 Mol/Mol Acetoacetamid einsetzt.

3. Verfahren nach Anspruch 1 oder 2, dadurch gekennzeichnet, daß man das $SO_3$ in Form von $SO_3$-abspaltenden reaktiven $SO_3$-Derivaten verwendet.

4. Verfahren nach Anspruch 3, dadurch gekennzeichnet, daß man das $SO_3$ in Form von reaktiven Addukten von $SO_3$ an tertiäre Amine verwendet.

5. Verfahren nach einem oder mehreren der Ansprüche 1 bis 4, dadurch gekennzeichnet, daß man als inertes anorganisches Lösungsmittel flüssiges $SO_2$ und als inertes organisches Lösungsmittel mindestens ein Lösungsmittel aus der folgenden Reihe verwendet :

halogenierte aliphatische Kohlenwasserstoffe,
Kohlensäureester mit niederen aliphatischen Alkoholen,
Nitroalkane,
Pyridin und alkylsubstituierte Pyridine und
aliphatische Sulfone.

6. Verfahren nach Anspruch 5, dadurch gekennzeichnet, daß man als organisches Lösungsmittel mindestens ein Lösungsmittel aus der folgenden Reihe verwendet : halogenierte aliphatische Kohlenwasserstoffe mit bis zu 4 C-Atomen, Kohlensäureester mit Methanol oder Ethanol, Nitroalkane mit bis zu 4 C-Atomen, Collidin und Sulfolan.

7. Verfahren nach Anspruch 6, dadurch gekennzeichnet, daß man als inerte organische Lösungsmittel mindestens eines der folgenden Lösungsmittel verwendet : Ethylenchlorid, Chloroform, 1,2-Dichlorethan, Dimethylcarbonat und Nitromethan.

8. Verfahren nach Anspruch 6, dadurch gekennzeichnet, daß man als inertes organisches Lösungsmittel Dichlormethan verwendet.

9. Verfahren nach einem oder mehreren der Ansprüche 1 bis 8, dadurch gekennzeichnet, daß man die Umsetzung bei einer Temperatur zwischen — 70 und + 180 °C, vorzugsweise bei einer Temperatur zwischen — 40 und + 90 °C durchführt.

10. Verfahren nach einem oder mehreren der Ansprüchen 1 bis 9, dadurch gekennzeichnet, daß man als Base für die Neutralisation des 6-Methyl-3,4-dihydro-1,2,3-oxathiazin-4-on-2,2-dioxids eine Kaliumbase verwendet.

## Claims

1. A process for the preparation of 6-methyl-3,4-dihydro-1,2,3-oxathiazin-4-one 2,2-dioxide and its non-toxic salts, starting from an acetoacetyl compound, which comprises reacting acetoacetamide with at least twice the molar amount of $SO_3$, where appropriate in an inert inorganic or organic solvent, and then, where appropriate, also neutralizing with a base the 6-methyl-3,4-dihydro-1,2,3-oxathiazin-4-one 2,2-dioxide which is produced in the form of the acid in this reaction.

2. The process as claimed in claim 1, wherein the $SO_3$ is used in an amount of from 2 to 20 mole/mole of acetoacetamide, preferably in an amount of from 4 to 10 mole/mole of acetoacetamide.

3. The process as claimed in claim 1 or 2, wherein the $SO_3$ is used in the form of $SO_3$-eliminating reactive $SO_3$ derivatives.

4. The process as claimed in claim 3, wherein the $SO_3$ is used in the form of reactive adducts of $SO_3$ onto tertiary amines.

5. The process as claimed in one or more of claims 1 to 4, wherein the inert inorganic solvent used is liquid $SO_2$ and the inert organic solvent used is at least one solvent from the following list :

halogenated aliphatic hydrocarbons,
esters of carbonic acid with lower aliphatic alcohols,
nitroalkanes,
pyridine and alkyl-substituted pyridines, and
aliphatic sulfones.

6. The process as claimed in claim 5, wherein the organic solvent used is at least one of the solvents from the following list : halogenated aliphatic hydrocarbons having up to 4 carbon atoms, esters of carbonic acid with methanol or ethanol, nitroalkanes having up to 4 carbon atoms, collidine and sulfolane.

7. The process as claimed in claim 6, wherein the inert organic solvent used is at least one of the following solvents : ethylene chloride, chloroform, 1,2-dichloroethane, dimethyl carbonate and nitromethane.

8. The process as claimed in claim 6, wherein the inert organic solvent used is dichloromethane.

9. The process as claimed in one or more of claims 1 to 8, wherein the reaction is carried out at a temperature between — 70 and + 180 °C, preferably at a temperature between — 40 and + 90 °C.

10. The process as claimed in one or more of claims 1 to 9, wherein the base used for the neutralization of the 6-methyl-3,4-dihydro-1,2,3-oxathiazin-4-one, 2,2-dioxide is a potassium base.

## Revendications

1. Procédé pour la préparation du dioxyde-2,2 de méthyl-6 dihydro-3,4 oxathiazine-1,2,3 one-4 et de ses sels non toxiques, à partir d'un dérivé acétoacétylique, caractérisé en ce qu'on fait réagir l'acétoacétamide sur une quantité au moins 2-molaire de $SO_3$, éventuellement dans un solvant organique ou inorganique inerte, et qu'on neutralise, éventuellement encore à l'aide d'une base, le dioxyde-2,2 de méthyl-6 dihydro-3,4 oxathiazine-1,2,3 one-4 qui se forme alors sous forme acide.

2. Procédé selon la revendication 1, caractérisé en ce qu'on utilise le $SO_3$ en une quantité de 2 à 20 moles/mole d'acétoacétamide, de préférence en une quantité de 4 à 10 moles par mole d'acétoacétamide.

3. Procédé selon la revendication 1 ou 2, caractérisé en ce qu'on utilise le $SO_3$ sous la forme de dérivés réactifs de $SO_3$ pouvant éliminer le $SO_3$.

4. Procédé selon la revendication 3, caractérisé en ce qu'on utilise le $SO_3$ sous la forme de produits d'addition réactifs de $SO_3$ sur des amines tertiaires.

5. Procédé selon l'une ou plusieurs des revendications 1 à 4, caractérisé en ce qu'on utilise comme solvant inorganique inerte du $SO_2$ liquide, et comme solvant organique inerte au moins un solvant de la série suivante :

hydrocarbures aliphatiques halogénés,
esters d'un acide carboxylique et d'alcools aliphatiques inférieurs,
nitroalcanes,

pyridine et pyridines alkyl-substituées, et
sulfones aliphatiques.

6. Procédé selon la revendication 5, caractérisé en ce qu'on utilise comme solvant organique au moins un solvant de la série suivante : hydrocarbures aliphatiques halogénés ayant jusqu'à 4 atomes de carbone, esters d'un acide carboxylique avec le méthanol ou l'éthanol, nitroalcanes ayant jusqu'à 4 atomes de carbone, collidine et sulfolanne.

7. Procédé selon la revendication 6, caractérisé en ce qu'on utilise comme solvants organiques inertes au moins l'un des solvants suivants : chlorure d'éthylène, chloroforme, dichloro-1,2 éthane, carbonate de diméthyle, et nitrométhane.

8. Procédé selon la revendication 6, caractérisé en ce qu'on utilise comme solvant organique inerte le dichlorométhane.

9. Procédé selon l'une ou plusieurs des revendications 1 à 8, caractérisé en ce qu'on met en œuvre la réaction à une température comprise entre — 70 et + 180 °C, de préférence à une température comprise entre — 40 et + 90 °C.

10. Procédé selon l'une ou plusieurs des revendications 1 à 9, caractérisé en ce qu'on utilise comme base destinée à la neutralisation du dioxyde-2,2 de méthyl-6 dihydro-3,4 oxathiazine-1,2,3 one-4 une base potassique.